(19) Europäisches Patentamt
European Patent Office
Office européen des brevets

(11) Veröffentlichungsnummer: **0 182 224**
**B1**

(12) # EUROPÄISCHE PATENTSCHRIFT

(45) Veröffentlichungstag der Patentschrift: **25.10.89**

(51) Int. Cl.⁴: **C 07 D 211/14**, C 07 D 405/06, A 01 N 43/40

(21) Anmeldenummer: **85114233.1**

(22) Anmeldetag: **08.11.85**

(54) **Am Stickstoff substituierte 4-(p-tert.Butyl-phenyl)-3-methyl-piperidine, dessen quartäre Salze sowie deren Anwendung als Fungizide.**

(30) Priorität: **16.11.84 DE 3441927**

(43) Veröffentlichungstag der Anmeldung: **28.05.86 Patentblatt 86/22**

(45) Bekanntmachung des Hinweises auf die Patenterteilung: **25.10.89 Patentblatt 89/43**

(84) Benannte Vertragsstaaten: **AT BE CH DE FR GB IT LI NL SE**

(56) Entgegenhaltungen:
**DE-A-2 752 135**
**US-A-3 468 885**
**US-A-3 821 234**

(73) Patentinhaber: **BASF Aktiengesellschaft, Carl-Bosch- Strasse 38, D-6700 Ludwigshafen (DE)**

(72) Erfinder: **Himmele, Walter, Dr., Eichenweg 14, D-6909 Walldorf (DE)**
Erfinder: **Buschmann, Ernst, Dr., Georg- Ludwig-Krebs- Strasse 10, D-6700 Ludwigshafen (DE)**
Erfinder: **Sauter, Hubert, Dr., Neckarpromenade 20, D-6800 Mannheim 1 (DE)**
Erfinder: **Pommer, Ernst- Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft wertvolle neue Piperidinderivate, deren 4-Stellung mit einem p-tert.Butylphenylrest und deren 3-Stellung mit einer Methylgruppe substituiert ist und die am Stickstoff verschiedene Reste tragen, sowie die quartären Salze dieser Piperidine und diese enthaltende Fungizide. Die neuen Verbindungen besitzen hervorragende fungizide Wirkung und eignen sich zur Bekämpfung von Pilzen.

Es ist bekannt, N-Tridecyl-2,6-dimethyl-morpholin (US-A-3 468 885) für die Bekämpfung von Pilzen zu verwenden. Seine Wirkung ist bei geringen Aufwandmengen ungenügend.

Es wurde nun gefunden, das am Stickstoff substituierte Piperidine, deren 4-Stellung mit einem p-tert.Butylphenylrest und deren 3-Stellung mit einer Methylgruppe substituiert ist, mit der allgemeinen Formel I

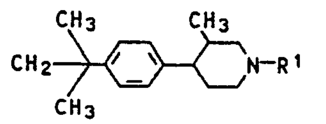

(I)

und die quartären Salze dieser Piperidine mit der allgemeinen Formel II

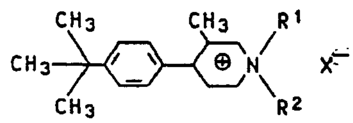

(II)

und die pflanzenverträglichen Salze der Piperidinverbindung der Formel I, eine ausgezeichnete fungizide Wirkung besitzen.

$R^1$ bedeutet einen Alkylrest mit 1 bis 10 C-Atomen (Methyl, Ethyl, Propyl, Butyl, Pentyl, Hexyl), einen Alkenylrest mit 2 bis 6 C-Atomen (Allyl, Methylallyl), einen Alkinylrest mit 3 bis 4 C-Atomen (Propargyl), wobei der Alkenylrest substituiert sein kann durch Phenyl oder Methoxyphenyl und in dem Alkylrest 1 bis 3 $CH_2$-Gruppen durch -O- ersetzt sein können und der Alkylrest substituiert sein kann durch Cycloalkyl mit 5 bis 7 C-Atomen (Cyclohexyl), Cycloalkenyl mit 5 bis 7 C-Atomen (Cyclohexenyl), Tetrahydropyran, Dioxan, Bicycloalkyl mit 8 bis 14 C-Atomen, Tricycloalkyl mit 8 bis 14 C-Atomen, Cycloalkoxy mit 5 bis 7 C-Atomen (Cyclohexyloxy), Phenyl, Phenoxy, wobei die cyclischen Reste ihrerseits wieder substituiert sein können durch Alkyl mit 1 bis 6 C-Atomen (Methyl, Ethyl, Propyl, Butyl), Alkoxy mit 1 bis 4 C-Atomen (Methoxy) oder Methylendioxy,

$R^2$ bedeutet einen Alkylrest mit 1 bis 4 C-Atomen (Methyl, Ethyl, Propyl, Butyl) oder einen Alkenylrest mit 2 bis 4 C-Atomen (Allyl, Methylallyl)

X bedeutet einen pflanzenverträglichen Säurerest.

Die Reste $R^1$ und $R^2$ können gleich oder verschieden sein.

Die fungizide Wirkung der neuen Verbindungen wird durch die Struktur der Piperidinverbindungen bestimmt, so daß die Salze beliebige pflanzenverträgliche Säurereste enthalten können, beispielsweise Reste der folgenden Säuren: Salzsäure, Bromwasserstoffsäure, Jodwasserstoffsäure, Schwefelsäure, Phosphorsäure, Salpetersäure, Ameisensäure, Essigsäure, Propionsäure, Benzolsulfonsäure, Dodecylbenzolsulfonsäure.

Am Piperidinring können die beiden Substituenten in 3- und 4-Stellung cis- oder trans-ständig sein. Eine Reindarstellung der trans- oder der cis-Form der neuen Verbindungen kann durch Fraktionierung der am Stickstoff nicht substituierten Piperidinverbindung und anschließende Einführung des Restes am Stickstoff erfolgen. Die Verbindungen enthalten in dem Kohlenstoffatomen 3 und 4 des Piperidinringes zwei asymmetrische Kohlenstoffatome. Sie kommen daher in Form ihrer optischen Isomeren und deren Enantiomerengemische vor. Die Gemische können in üblicher Weise in die einzelnen Isomeren aufgetrennt werden. Die Erfindung umfaßt sowohl die cis- und trans-Isomeren und die optischen Isomeren der neuen Verbindungen als auch ihre Mischungen.

Die Synthese der Verbindungen der allgemeinen Formel I kann ausgehend vom 4-tert.-Butyl-benzaldehyd durch Kondensation mit Propionaldehyd zum 3-(4'-tert.-Butylphenyl)-2-methyl-2-propen-al III.

**Stufe 1**

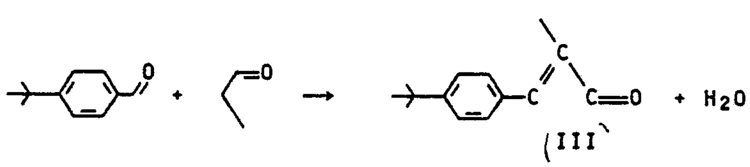

(III)

mit Hilfe von basischen Katalysatoren erfolgen.

Verbindung III kann mit Vinylisobutylether IV zum 4-(tert.Butylphenyl)-3-methyl-6-isobutoxy-5,6-dihydropyran (V) kondensiert werden.

**Stufe 2**

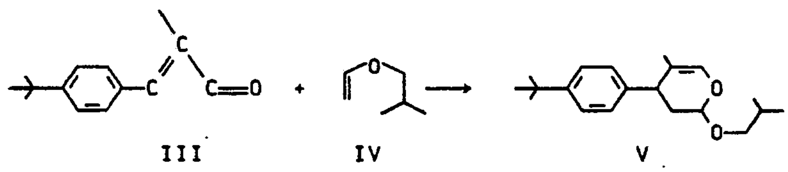

III IV V

Verbindung V kann mit Ameisensäure und Wasser zum 2-Methyl-3-(p-tert.-butylphenyl)-1,5-pentandial VI umgesetzt werden.

**Stufe 3**

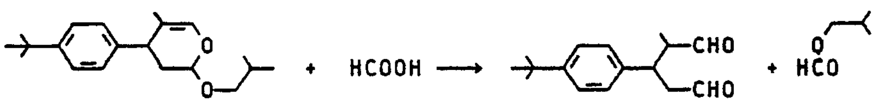

V VI

Das 2-Methyl-3-(p-tert.-butylphenyl)-1,5-pentandial VI kann mit Ammoniak und Wasserstoff in Gegenwart von Hydrierkatalysatoren zum 4-(p-tert.-Butylphenyl)-3-methylpiperidin VII hydriert werden.

**Stufe 4**

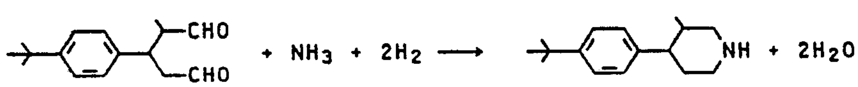

VI (VII)

Die Einführung des Restes R1 in die Verbindung VII kann mit den üblichen Methoden der Alkylierung von sekundären Aminen erreicht werden. Hierzu eignet sich insbesondere die Methode nach Leuckardt-Wallach (Houben-Weyl, Methoden der organischen Chemie, Band 11/2, S. 331, Georg Thieme Verlag Stuttgart, 1958).

Geeignet für eine Einführung eines der unter $R^1$ genannten Reste, ist auch die Hydrierung (beispielsweise mit Ameisensäure) eines Enamins E das aus der Verbindung VII und einer entsprechenden Carbonylverbindung synthetisiert wurde.

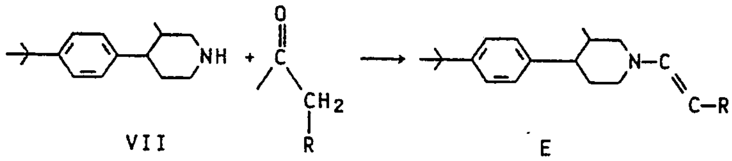

VII R E

Die Reinigung der Verbindung VII kann durch fraktionierte Destillation erfolgen. Diese Fraktionierung kann so ausgeführt werden, daß eine Trennung der stereoisomeren Formen, die auf die Stellung der beiden Substituenten in der 3- und 4-Stellung am Piperidinring zurückzuführen ist, weitgehend erreicht werden kann. Das trans-ständige Produkt VIIa siedet im allgemeinen niedriger als das cis-ständige Produkt VIIb.

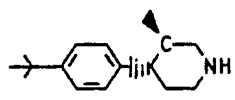

VIIa

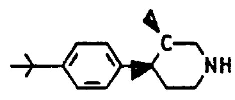

VIIb

Bei den unten beschriebenen Herstellungsbedingungen bildet sich zum überwiegenden Teil die trans-Form VIIa (etwa 75 %).

Eine weitere Synthesemöglichkeit für Verbindungen gemäß allgemeinen Formel I und II besteht in der Umsetzung von Verbindung VI mit Aminen zu 1,4-Dihydropyridinen VIII und anschließender Hydrierung der Verbindungen vom Typ VIII zu den Verbindungen von Typ I.

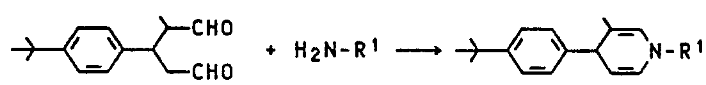

VI VIII

Eine Reinigung der Verbindungen VIII vor der Hydrierung wird zweckmäßig unterlassen und die Hydrierung mit dem rohen Kondensationsprodukt durchgeführt, um Verluste durch Abspaltung des Restes $R^1$ unter Ausbildung der Pyridinstruktur zu vermeiden.

Die Reinigung der Verbindungen der allgemeinen Formel I kann in den meisten Fallen durch eine Kurzwegdestillation erfolgen. Bei Verbindungen der allgemeinen Formel I mit einem größeren Rest $R^1$, bei denen zu hohe Siedetemperaturen zu erwarten sind, kann auch eine Reinigung über Salzbildung und anschließende Freisetzung der Base erreicht werden.

Die Verbindungen der allgemeinen Formel I sind meist dickflüssige Öle. In einigen Fällen konnten kristalline Produkte erhalten werden. Die Umsetzung der Verbindungen der allgemeinen Formel I zu den Verbindungen der allgemeinen Formel II erfolgt durch Umsetzung mit einer Verbindung der allgemeinen Formel $R^2X$ in üblicher Weise.

**Vorschrift 1**

für die Synthese von 4-(p-tert.-Butylphenyl)-3-methyl-6-isobutoxy-5,6-dihydropyran V

In einen 10-l-Rührautoklav werden 3000 g 3-(4-tert.-Butyl phenyl)-2-methyl-prop-2-en-al III und 2000 g Vinylisobutylether IV eingefüllt. Dem Reaktionsgemisch wird zur Stabilisierung 5 g Hydrochinon zugesetzt. Der Autoklav wird nach dem Verschließen mit Stickstoff gespült. Der Autoklav wird danach auf 220°C aufgeheizt und 18 Stunden bei diesen Bedingungen gehalten und danach entspannt und geöffnet. Das Reaktionsprodukt (4855 g) besteht zu etwa 39,6 % aus dem gewünschten Reaktionsprodukt V. Bei der Aufarbeitung des Reaktionsgemisches durch Fraktionierung in einer Laborkolonne mit 10 Siebböden werden von den nach GC(Gaschromatographie)-Analyse im Rohgemisch vorhandenen 1923 g insgesamt 1379 g der Verbindung V mit einem Siedepunkt von 135 bis 146°C bei einem Druck von 2 mbar erhalten. Das entspricht einer Ausbeute von 23 %, bezogen auf das eingesetzte Produkt III. 1745 g Produkt III, das zum Teil Produkt V enthält, werden wiedergewonnen. Die zuletzt genannten Anteile werden erneut zur Umsetzung mit Vinylisobutylether verwendet.

**Vorschrift 2**

für die Synthese von 3-(4'-tert.-Butylphenyl)-2-methyl-1,5-pentandial VI

In einem 2-l-Rührkolben, der mit Thermometer, Zutropfmöglichkeit und Trennaufsatz für Flüssigkeiten ausgestattet ist, werden 600 g Ameisensäure und 600 g Wasser vorgelegt. Das Gemisch wird auf 100°C aufgeheizt und bei dieser Temperatur im Verlauf von 6 Stunden 1200 g Produkt V zugetropft. Es destillieren 277 g Isobutylformiat ab. Nach weiteren 6 Stunden Reaktionszeit werden noch weitere 134 g Isobutylformiat erhalten. Dem Reaktionsgemisch werden nun 300 g Toluol zugesetzt und die Schichtentrennung abgewartet. Es bildet sich beim Stehen über Nacht eine untere Schicht (890 g), die aus Wasser/Ameisensaure besteht und eine obere Schicht von 1415 g, die das Produkt VI enthält, aus.

Die organische Phase wird in einer Kurzwegdestillationsapparatur einer diskontinuierlichen Fraktionierung unterworfen. Bei einem Druck von 0,2 mbar destillieren zwischen 150 bis 165°C 712 g Produkt VI über. Diese stellen ein leicht gelbliches Öl dar und werden zwecks Stabilisierung mit 356 g Tetrahydrofuran versetzt. Die Ausbeute an Produkt VI, bezogen auf eingesetztes Produkt V, liegt bei 60 %.

**Vorschrift 3**

für die Synthese von 4-(4'-tert.-Butylphenyl)-3-methyl-piperidin VII durch aminierende Hydrierung von Produkt VI.

In einem 5-l-Autoklav werden 625 g Methanol zusammen mit 100 g Raney-Kobalt vorgelegt. Der Autoklav wird druckdicht verschlossen und mit Stickstoff gespült. Danach werden 500 g Ammoniak (flüssig) eingepreßt. Der Autoklav wird anschließend auf 100°C aufgeheizt und ein Wasserstoffdruck von 150 bar eingestellt. Im Verlauf von 8 Stunden wird ein Gemisch von 800 g Produkt VI und 270 g Tetrahydrofuran in den Autoklav eingepreßt. Die Einpreßleitung wird durch Nachpressen von 100 g Tetrahydrofuran gespült. Nach dem Einpressen wird das Reaktionsgemisch noch weitere 6 Stunden unter Rühren unter den genannten Reaktionsbedingungen gehalten. Verbrauchter Wasserstoff wird in Abständen von 2 Stunden nachgepreßt, um den Wasserstoffdruck beizubehalten.

Danach wird die Heizung des Autoklav abgestellt und der Autoklav nach dem Abkühlen auf eine Temperatur von 60°C vorsichtig entspannt. Dabei entweicht neben dem nicht verbrauchten Wasserstoff ein Teil des Ammoniaks. Nach dem Entspannen läßt sich das Reaktionsgemisch (2112 g) aus dem Autoklav entnehmen. Durch Filtration wird es vom Katalysator befreit.

Bei einem Druck von 50 mbar werden Methanol, Tetrahydrofuran und das bei der Reaktion entstandene Wasser abdestilliert. Bei der fraktionierenden Destillation des rohen Amingemisches werden 576 g Produkt VII in einem Siedebereich zwischen 125 bis 136°C bei einem Druck von 1 mbar erhalten. Die gaschromatographische Analyse zeigt, daß etwa 85 % als trans-Form VIIa und 15 % als cis-Form VIIb vorliegen. Bezogen auf das Produkt VI liegt die Ausbeute an VII bei 77 %. Sowohl die trans-angereicherte Fraktion als auch die cisangereicherte Fraktion kristallisieren beim Stehen über Wochen zum Teil aus.

**Beispiel 1**

Alkylierung des 4-(4'-tert.-Butylphenyl)-3-methyl-piperidins, Synthese von N-Hexahydrobenzyl-4-(4'-tert.-butylphenyl)-3-methyl-piperidin IX (Verbindung Nr. 7 in Tabelle 1)

30 g 4-(4'-tert.-Butylphenyl)-3-methyl-piperidin VII wurden mit 17,5 g Hexahydrobenzaldehyd vermischt. Nach zweistündigem Stehen wurden 36 g Ameisensäure zugegeben. Dieses Reaktionsgemisch wurde 12 Stunden am Rückfluß erhitzt.

Durch Fraktionierung mittels einer Kurzwegdestillation werden die folgenden Fraktionen gewonnen:

| | | |
|---|---|---|
| 1 bis 185°C/5 mbar | 4 g | Nach GC-Analyse circa 86 % Verbindung IX |
| 2 bis 190°C/5 mbar | 31 g | " |
| Destillationsrückstand | 4 g | " |

Die NMR- und IR-Spektren bestätigen das Vorliegen der Verbindung IX. Als Verunreinigung liegt noch das N-Formylprodukt von VII vor.

**Beispiel 2**

Synthese des 1-(3',5',5'-Trimethyl-hexyl-1')-4-(4'-tert.-butylphenyl)-3-methyl-piperidins X (Verbindung Nr. 21 in Tabelle 1)

28,9 g VIIa wurden mit 23,7 g 3,5,5-Trimethyl hexanal vermischt. Dabei trat Erwärmung auf. Nach zwei Stunden wurden 46 g Ameisensäure zugegeben. Dieses Reaktionsgemisch wurde 12 Stunden am Rückfluß erhitzt. Die Isolierung von X erfolgte durch Fraktionierung mittels einer Kurzwegdestillation. Neben Vorlauf und einem kleinen Zwischenlauf bis zu einer Übergangstemperatur von 163°C/2 mbar von 5 g gehen bei 163 bis 174°C/2 mbar 41 g über. Der Destillationsrückstand beträgt 4 g. Nach der gaschromatographischen Analyse besteht die Hauptmenge (41 g) zu 89 % aus Verbindung X. Die NMR- und IR-Spektren bestätigen das Vorliegen der Verbindung X.

**Beispiel 3**

Synthese von 1-(3',5',5'-Trimethyl-hexyl-1')-3-methyl-4-(4'-tert.-butylphenyl)-1-methyl-piperidinium-jodid XI

11 g der Verbindung X wurden in 20 g Aceton gelöst und mit 4 g Methyljodid versetzt. Das Gemisch erwärmte sich schwach. Nach einigen Minuten schied sich das quartäre Piperidiniumsalz als feinkristallines Pulver ab. Durch Absaugen und Trocknen wurden 8 g von der Verbindung XI mit einem Fp. von 280°C gewonnen.

Das NMR-Spektrum bestätigt das Vorliegen der Verbindung XI.

In Tabelle 1 sind die am Stickstoff substituierten Derivate des 4-(4'-tert.-Butylphenyl)-3-methyl-piperidins (allgemeine Formel I) aufgeführt. Die Synthese dieser Verbindungen erfolgte nach der unter Beispiel 1 und Beispiel 2 angegebenen Arbeitsweise.

In Tabelle 2 sind Verbindungen der allgemeinen Formel II zusammengestellt. Diese Verbindungen wurden aus Verbindungen der allgemeinen Formel I durch Umsetzung mit Methyljodid gewonnen. Es wurde entsprechend Beispiel 3 gearbeitet.

In Tabelle 3 sind Substanzen der allgemeinen Formel II aufgeführt. Diese Substanzen wurden aus Verbindungen der allgemeinen Formel I durch Umsetzung mit Allylbromid erhalten. Gearbeitet wurde nach der unter Beispiel 3 erläuterten Arbeitsweise.

**Tabelle 1**

| Verbindung Nr. | $R^1$ (allgemeine Formel I) | Konfiguration in 3- und 4-Stellung | Physikalische Daten Siedepunkt in °C/mbar |
|---|---|---|---|
| 1 | n-Propyl | - | - |
| 2 | i-Propyl | - | - |
| 3 | n-Butyl | - | - |
| 4 | 2-Methyl-propyl | trans | 160 - 170/5 |
| 5 | 3-Methyl-butyl | trans | 143 - 146/3 |
| 6 | Benzyl | trans | 190 - 200/2 |
| 7 | Hexahydrobenzyl | trans | 185 - 190/5 |
| 8 | Methyl | 70 % trans/30 % cis | 118 - 130/0,2 |
| 9 | 4-Methoxybenzyl | mehr als 95 % trans | 186/2 |
| 10 | 3-(4-tert.-Butylphenyl)-2-methyl-propyl | mehr als 95 % trans | 225/2 |
| 11 | Ethyl | - | - |
| 12 | 3-Phenyl-2-propenyl | mehr als 95 % trans | 220 - 230/2 |
| 13 | 2-Ethyl-hexyl | mehr als 95 % trans | 174 - 178/2 |
| 14 | 4-tert.-Butyl-benzyl | mehr als 95 % trans | 188/2 |
| 15 | 2-Phenyl-propyl | mehr als 95 % trans | 180/2 |
| 16 | 3-Phenyl-butyl | mehr als 95 % trans | 196/2 |
| 17 | 3-(4-Methyl-3-cyclo-hexenyl)-butyl | mehr als 95 % trans | 163/0,8 |
| 18 | 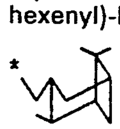 | mehr als 95 % trans | 160 - 162/1 |
| 19 | (4-Methyl-tetrahydro-pyran-3-yl)-methyl | mehr als 95 % trans | 167 - 172/1 |
| 20 | 5,5,3-Trimethylhexyl | mehr als 90 % cis | 172/1 |

| | | | |
|---|---|---|---|
| 21 | 5,5,3-Trimethylhexyl | mehr als 96 % trans | 174 - 176/2 |
| 22 | 4,4-Dimethyl-pentyl | mehr als 96 % trans | 154/1 |
| 23 | 4,4-Dimethyl-pentyl | mehr als 90 % cis | 178/3 |
| 24 | 2-(3,4-Dimethoxy-phenyl)-butyl | mehr als 95 % trans | 196 - 210/2 |
| 25 | 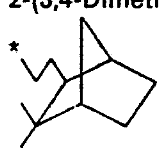 | mehr als 95 % trans | 190 - 194/1 |
| 26 | 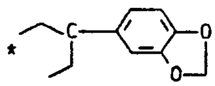 | mehr als 95 % trans | 200 - 212/2 |
| 27 | 3-(4-tert.-Butyl-cyclo-hexyl)-2-methyl-propyl | mehr als 95 % trans | 222 - 224/2 |
| 28 | 2-Phenyl-hexyl | mehr als 95 % trans | 188 - 200/2 |
| 29 | 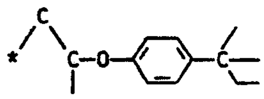 | mehr als 95 % trans | 210 - 215/2 |
| 30 | 2-Phenoxy-propyl | mehr als 95 % trans | 178 - 183/2 |
| 31 | 2-(3-Methyl-phenoxy)-propyl | mehr als 95 % trans | 210/2 |
| 32 | 2-(3-Methyl-phenoxy)-propyl | mehr als 95 % trans | 208 - 211/2 |
| 33 | 2-(4-Methoxy-phenyl)-butyl | mehr als 95 % trans | 212/1 |
| 34 | 2-Phenyl-2-methyl-propyl | mehr als 95 % trans | 166 - 170/1 |
| 35 | 3,3,2-Trimethyl-butyl | 70 % trans/30 % cis | 142 - 150/1 |
| 36 | 2-(Tetrahydropyran-4-yl)-ethyl | mehr als 95 % trans | 196/2 |
| 37 | 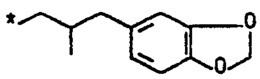 | mehr als 95 % trans | 197 - 218/2 |
| 38 | 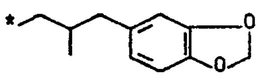 | mehr als 95 % cis | 220 - 226/2 |
| 39 | (Tetrahydropyran-2-yl)-methyl | mehr als 98 % trans | 174 - 184/2 |
| 40 | (Tetrahydropyran-3-yl)-methyl | mehr als 98 % trans | 200 - 220/ |
| 41 | (1,4-Dioxan-2-yl)-methyl | mehr als 98 % trans | 180 - 185/2 |
| 42 | 2-Methyl-3,6-dioxa-octyl | mehr als 95 % cis | 170 - 176/1 |

**Tabelle 2**

| Verbindung Nr. | $R^1$ | $R^2$ | Konfiguration in 3- und 4-Stellung | Fp. in °C Zers. = Zersetzung | Anion |
|---|---|---|---|---|---|
| 43 | Methyl | Methyl | 65 trans/35 cis | Fp. 200 - 206 | Jodid |
| 44 | Methyl | 3-(4-tert.-Butyl-phenyl)-2-methyl-propyl | mehr als 95 % trans | Fp. 236 - 237 | Jodid |
| 45 | Methyl | 3-(4-tert.-Butyl-phenyl)-2-methyl-propyl | mehr als 95 % trans | Fp. 155 - 180 | Hydrogen-sulfat |
| 46 | Methyl | 2-Methyl-butyl | mehr als 95 % trans | Fp. 223 - 232 | Jodid |
| 47 | Methyl | 2-Methyl-propyl | mehr als 95 % trans | Fp. 228 - 234 | Jodid |
| 48 | Methyl | 3-Phenyl-2-propenyl | mehr als 95 % trans | Fp. 186 - 188 | Jodid |
| 49 | Methyl | 2-Ethyl-hexyl | mehr als 95 % trans | Fp. 153 - 155 | Jodid |
| 50 | Methyl | 2-(4-Methoxy-phenyl)-butyl | mehr als 95 % trans | mehr als 260 | Jodid |
| 51 | Methyl | 2-Phenyl-propyl | mehr als 95 % trans | 280 Zers. | Jodid |
| 52 | Methyl | 5,5,3-Trimethyl-hexyl | mehr als 95 % trans | 250 Zers. | Jodid |
| 53 | Methyl | 4-tert.-Butyl-benzyl | mehr als 95 % trans | Fp. 224 - 230 | Jodid |
| 54 | Methyl | 3-Phenyl-butyl | mehr als 95 % trans | Fp. 157 - 172 | Jodid |
| 55 | Methyl | 2-Phenyl-hexyl | mehr als 95 % trans | Fp. 240 - 247 | Jodid |
| 56 | Methyl | 2-Phenyl-2-methyl | mehr als 95 % trans | mehr als 260 Zers. | Jodid |
| 57 | Methyl | 5,5,3-Trimethyl-hexyl | mehr als 90 % cis | 250 Zers. | Jodid |

| | | | | | |
|---|---|---|---|---|---|
| 58 | Methyl | 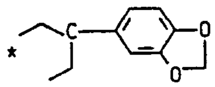 | mehr als 95 % trans | mehr als 260 Zers. | Jodid |
| 59 | Methyl | 4,4-Dimethyl-pentyl | mehr als 90 % cis | Fp. 235 - 242 | Jodid |
| 60 | Methyl | 3-(4-tert.Butyl-cyclohexyl)-2-methyl-propyl | mehr als 95 % trans | mehr als 260 Zers. | Jodid |
| 61 | Methyl | 2-Phenoxy-propyl | mehr als 95 % trans | Fp. 212 - 224 | Jodid |
| 62 | Methyl | 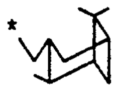 | mehr als 95 % trans | mehr als 260 Zers. | Jodid |
| 63 | Methyl | 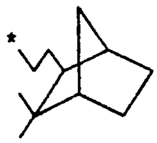 | mehr als 95 % trans | mehr als 260 Zers. | Jodid |
| 64 | Methyl | 5,5,3-Trimethyl-hexyl | mehr als 95 % trans | mehr als 260 Zers. | Jodid |
| 65 | Methyl | Methyl | mehr als 95 % trans | Fp. 228 - 233 | Jodid |
| 66 | Methyl | 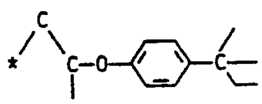 | mehr als 95 % trans | mehr als 260 Zers. | Jodid |
| 67 | Methyl | 2-(4-tert.-Butyl-phenoxy)-propyl | mehr als 95 % trans | mehr als 260 Zers. | Jodid |

**Tabelle 3**

| Verbindung. Nr. | $R^1$ | $R^2$ | Konfiguration in 3- und 4-Stellung | Fp. in °C Zers. = Zersetzung | Anion |
|---|---|---|---|---|---|
| 68 | Allyl | Methyl | 67 % trans/33 % cis | Fp. 198 - 212 | Bromid |
| 69 | Allyl | 3-Methyl-butyl | mehr als 95 % trans | Fp. 229 - 231 | Bromid |
| 70 | Allyl | 2-Methyl-propyl | mehr als 95 % trans | Fp. 225 - 229 | Bromid |
| 71 | Allyl | 3-Phenyl-2-propenyl | mehr als 95 % trans | Fp. 146 - 150 | Bromid |
| 72 | Allyl | 5,5,3-Trimethyl-hexyl | mehr als 95 % trans | Fp. 191 - 208 | Bromid |
| 73 | Allyl | 3-Phenyl-butyl | mehr als 95 % trans | Fp. 196 | Bromid |
| 74 | Allyl | 2-Phenyl-hexyl | mehr als 95 % trans | Fp. 180 - 187 | Bromid |

Die neuen Verbindungen zeichnen sich, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse - wie Gurken, Bohnen und Kürbisgewächse.

Die neuen Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora musae an Bananen,
Pseudocercosporella herpotrichoides an Weizen, Gerste,

Pyricularia oryzae an Reis,
Hemileia vastatrix an Kaffee,
Alternaria solani an Kartoffeln, Tomaten,
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Staube, Pulver, Plasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Ketone (z. B. Cyclohexanon). Amine (z. B. Ethanolamin, Dimethylformamid) und Wasser; Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel wie nichtionogene und anionische Emulgatoren (z. B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.-% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die neuen Verbindungen haben auch eine sehr gute Wirksamkeit gegenüber hautpathogenen Pilzen, insbesondere Candida albicans.

Die Verbindungen können allein oder zusammen mit anderen bekannten Wirkstoffen, insbesondere Antibiotika, angewendet werden.

Die Herstellung der chemotherapeutischen Mittel oder Zubereitungen mit üblichen festen, halbfesten oder flüssigen Trägerstoffen oder Verdünnungsmitteln und den üblicherweise verwendeten pharmazeutisch-technischen Hilfsstoffen entsprechend der gewünschten Applikationsart mit einer zur Anwendung geeigneten Dosierung erfolgt in üblicher Weise, insbesondere durch Vermischen (vgl. H. Sucker et al., Pharmazeutische Technologie, Thieme-Verlag, Stuttgart, 1978).

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions etc. in Betracht.

Die therapeutisch wirksame Verbindung ist in pharmazeutischen Zubereitungen vorzugsweise in einer Konzentration von 0,5 bis 90 Gew.-% der Gesamtmischung vorhanden.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 4 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 19 werden in einer Mischung gelöst, die aus 80 Gewichtsteilen Xylol, 10 Gewichtsteilen des Anlagerungsproduktes von 8 bis 10 Mol Ethylenoxid an 1 Mol Öl-säure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 21 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 36 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 69 werden den mit 3 Gewichtsteilen des Natriumsalzes der Diisobutylnaphthalin-alpha-sulfonsäure, 10 Gewichtsteilen des Natriumsalzues einer Ligninsulfonsäure

aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 70 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung Nr. 19 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 6 werden mit 10 Gewichtsteilen Natriumsalz eines Phenolsulfonsäure-harnstoff-formaldehyd-Kondensates, 2 Gewichtsteilen Kieselgel und 48 Gewichtsteilen Wasser innig vermischt. Man erhält eine stabile wäßrige Dispersion. Durch Verdünnen mit Wasser erhält man eine wäßrige Dispersion.

IX. 20 Gewichtsteile der Verbindung Nr. 7 werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenolsulfonsäure-harnstoff-formaldehyd-Kondensates und 68 Gewichtsteilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschranken.

Fungizide, die mit den erfindungsgemäßen Verbindunger kombiniert weiden können, sind beispielsweise:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester

Heterocyclen, wie
2-Heptadecyl-2-imidazolin-acetat
2,4-Dichlor-6-(o-chloranilino)-s-triazin
0,0-Diethyl-phthalimidophosphonothioat
5-Amino-1-[bis-(dimethylamino)-phosphinyl]-3-phenyl-1,2,4-triazol
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid

8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl- 5,6-dihydro-4H-pyran-3-carbonsäureanilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäureanilid
2-Iod-benzoesäureanilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-[3-(3,5-Dimethyl-2-oxycyclohexyl)-2-hydroxyethyl]-gluatarimid Hexachlorbenzol
DL-Methyl-N-(2,6-dimethyl-phenyl)-N-furoyl-(2)-alaninat
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-[3,5-Dichlorphenyl-(5-methyl-5-methoxymethyl)]-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid
2-Cyano-[N-(ethylaminocarbonyl)-2-methoximino]-acetamid
1-[2-(2,4-Dichlorphenyl)pentyl]-1H-1,2,4-triazol
2,4-Difluor-alpha-(1H-1,2,4-triazolyl-1-methyl)-benzhydrylalkohol

Für die folgenden Versuche wurden als Vergleich die bekannten Wirkstoffe verwendet:
N-Tridecyl-2,6-dimethyl-morpholin (A) und das essigsaure Salz (Acetat) von A (B) verwendet.

**Anwendungsbeispiel 1**

Wirksamkeit gegen Weizenmehltau

Blätter von in Töpfen gewachsenen Weizenkeimlingen der Sorte Frühgold werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 7 Tagen wird das Ausmaß der Mehltauentwicklung ermittelt.

Das Ergebnis des Versuches zeigt, daß beispielsweise die Wirkstoffe 4, 19, 21, 36, 69 und 70 bei der Anwendung als 0,025 %-ige Spritzbrühe eine bessere fungizide Wirkung zeigten (beispielsweise 97 %) als die Wirkstoffe A und B (90 %).

**Anwendungsbeispiel 2**

Wirksamkeit gegen Gurkenmehltau

Blätter von in Töpfen gewachsenen Gurkenkeimlingen der Sorte "Chinesische Schlange" werden im Zweiblattstadium mit einer Sporensuspension des Gurkenmehltaus (Erysiphe cichoracearum) besprüht. Nach etwa 20 Stunden werden die Versuchspflanzen mit wäßriger Spritzbrühe. die 80 % Wirkstoff und 20 % Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22° C und 75 bis 80 % relativer Luftfeuchtigkeit aufgestellt. Zur Beurteilung der Wirksamkeit der neuen Stoffe wird das Ausmaß der Pilzentwicklung nach 21 Tagen ermittelt.

Das Ergebnis des Versuches zeigt, daß beispielsweise die Wirkstoffe 4, 6, 7, 9, 12, 13, 14, 15, 16, 19, 22, 23, 25, 30, 33, 36, 38, 46, 47, 48, 49, 52, 57, 59, 69, 70 und 71 bei der Anwendung als 0,025 %-ige Spritzbrühe eine bessere fungizide Wirkung zeigten (beispielsweise 97 %) als der Wirkstoff A (60 %).

**Anwendungsbeispiel 3**

Wirksamkeit gegen Weizenbraunrost

Blätter von in Töpfen gewachsenen Weizensämlingen der Sorte Frühgold werden mit Sporen des Braunrostes (Puccinia recondita) bestäubt. Danach werden die Töpfe für 24 Stunden bei 20 bis 22° C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22° C und 65 bis 70 % relativer Luftfeuchtigkeit aufgestellt. Nach 8 Tagen wird das Ausmaß der Rostpilzentwicklung auf den Blättern ermittelt.

Das Ergebnis des Versuches zeigt, daß beispielsweise die Wirkstoffe 4, 5, 6, 7, 15, 19, 20, 23, 36, 47 und 70 bei der Anwendung als 0.025 %-ige Spritzbrühe eine bessere fungizide Wirkung zeigten (beispielsweise 97 %) als die Wirkstoffe A und 8 (50 %).

**Anwendungsbeispiel 4**

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßriger Spritzbrühe, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24° C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise die Wirkstoffe 4, 6, 15, 16, 18, 25, 26, 29, 38, 44, 45, 48, 52, 53, 56, 57, 60, 62, 63, 66, 67, 72 und 74 bei der Anwendung als 0.05 %-ige Spritzbrühe eine bessere fungizide Wirkung zeigten (beispielsweise 97 %) als der Wirkstoff A (70 %).

**Anwendungsbeispiel 5**

Wirksamkeit gegen Candida albicans

0,1 ml einer Keimsuspension von Candida albicans in Caseinpepton-Sojabohnenmehlpepton-Lösung werden mit den zu prüfenden wäßrigen Wirkstoffverdünnungen gut vermischt. Nach einer Einwirkungszeit von 10 Minuten wird den Wirkstoffkeimgemischen jeweils 1 ml entnommen und zu 9 ml Inaktivierungsflüssigkeit (0,1 % Trypton + 0,85 % Natriumchlorid in aqua bidest. + 3 % Tween 80 + 0,3 % Lecithin + 0,1 % Cystein) zugesetzt. Nach 30-minutiger Kontaktzeit in der Inaktivierungsflüssigkeit werden aus den Ansätzen Proben von Caseinpepton-Sojabohnenmehl pepton-Agar in Petrischalen übertragen. Die Petrischalen werden anschließend 72 Stunden bei 37° C bebrütet und die Wirkstoffkonzentration ermittelt bei der kein Wachstum des Pilzes mehr erfolgt, d.h. es wird die Konzentration ermittelt, bei welcher der Pilz abgetötet wird. Die Wirkstoffe Nr. 60 und 66 bewirkten bei einer Konzentration von 6 ppm die Abtötung des Pilzes.

**Patentansprüche**

1. Piperidinderivat der allgemeinen Formel I

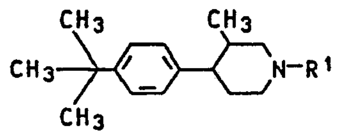

(I)

oder der allgemeinen Formel II

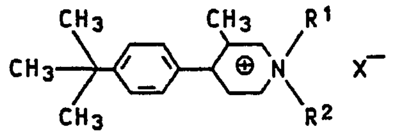

(II)

in der $R^1$ einen Alkylrest mit 1 bis 10 C-Atomen, einen Alkylenrest mit 2 bis 6 C-Atomen, einen Alkinylrest mit 3 bis 4 C-Atomen bedeutet, wobei der Alkenylrest substituiert sein kann durch Phenyl oder Methoxyphenyl und in dem Alkylrest 1 bis 3 $CH_2$-Gruppen durch -O- ersetzt sein können und der Alkylrest substituiert sein kann durch Cycloalkyl mit 5 bis 7 C-Atomen, Cycloalkenyl mit 5 bis 7 C-Atomen, Tetrahydropyran, Dioxan, Bicycloalkyl mit 8 bis 14 C-Atomen, Tricycloalkyl mit 8 bis 14 C-Atomen, Cycloalkoxy mit 5 bis 7 C-Atomen, Phenyl, Phenoxy, wobei die cyclischen Reste ihrerseits wieder substituiert sein können durch Alkyl mit 1 bis 6 C-Atomen, Alkoxy mit 1 bis 4 C-Atomen oder Methylendioxy,

$R^2$ einen Alkylrest mit 1 bis 4 C-Atomen oder einen Alkenylrest mit 2 bis 4 C-Atomen, bedeutet,

X einen pflanzenverträglichen Säurerest bedeutet

und die pflanzenverträglichen Salze dem Piperidinderivats der allgemeinen Formel I.

2. Piperidinderivat gemäß Anspruch 1 der allgemeinen Formel II, in der $R^2$ Methyl oder Allyl und $X^\ominus$ Jodid oder Bromid bedeutet.

3. Fungizid, enthaltend eine Verbindung gemäß Anspruch 1.

4. Fungizid, enthaltend einen festen, halbfesten oder flüssigen Trägerstoff und ein Piperidinderivat der allgemeinen Formel I

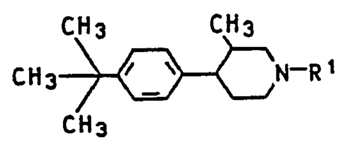

(I)

oder der allgemeinen Formel II

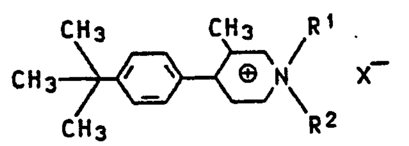

(II)

in der $R^1$ einen Alkylrest mit 1 bis 10 C-Atomen, einen Alkenylrest mit 2 bis 6 C-Atomen, einen Alkinylrest mit 3 bis 4 C-Atomen bedeutet, wobei der Alkenylrest substituiert sein kann durch Phenyl oder Methoxyphenyl und in dem Alkylrest 1 bis 3 $CH_2$-Gruppen durch -O- ersetzt sein können und der Alkylrest substituiert sein kann durch Cycloalkyl mit 5 bis 7 C-Atomen, Cycloalkenyl mit 5 bis 7 C-Atomen, Tetrahydropyran, Dioxan, Bicycloalkyl mit 8 bis 14 C-Atomen, Tricycloalkyl mit 8 bis 14 C-Atomen, Cycloalkoxy mit 5 bis 7 C-Atomen, Phenyl, Phenoxy, wobei die cyclischen Reste ihrerseits wieder substituiert sein können durch Alkyl mit 1 bis 6 C-Atomen, Alkoxy mit 1 bis 4 C-Atomen oder Methylendioxy,

$R^2$ einen Alkylrest mit 1 bis 4 C-Atomen oder einen Alkenylrest mit 2 bis 4 C-Atomen, bedeutet,

X einen pflanzenverträglichen Säurerest bedeutet oder ein pflanzenverträgliches Salz des Piperidinderivats der Formel I.

5. Verfahren zur Herstellung eines Fungizids, dadurch gekennzeichnet, daß man einen festen, halbfesten oder flüssigen Trägerstoff vermischt mit einer Verbindung gemäß Anspruch 1.

6. Verfahren zur Bekämpfung von Pilzen, außer in der Human- oder Veterinärmedizin, dadurch gekennzeichnet, daß man die Pilze oder die von Pilzbefall bedrohten Materialien, Pflanzen, Saatgüter oder Boden behandelt (mit einem Piperidinderivat der allgemeinen Formel I

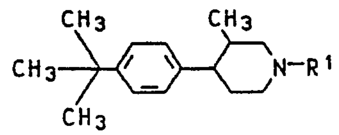

(I)

oder der allgemeinen Formel II

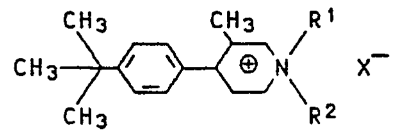

(II)

in der $R^1$ einen Alkylrest mit 1 bis 10 C-Atomen, einen Alkenylrest mit 2 bis 6 C-Atomen einen Alkinylrest mit 3 bis 4 C-Atomen bedeutet, wobei der Alkenylrest substituiert sein kann durch Phenyl oder Methoxyphenyl und in dem Alkylrest 1 bis 3 $CH_2$-Gruppen durch -O- ersetzt sein können und der Alkylrest substituiert sein kann durch Cycloalkyl mit 5 bis 7 C-Atomen, Cycloalkenyl mit 5 bis 7 C-Atomen, Tetrahydropyran, Dioxan, Bicycloalkyl mit 8 bis 14 C-Atomen, Tricycloalkyl mit 8 bis 14 C-Atomen, Cycloalkoxy mit 5 bis 7 C-Atomen, Phenyl, Phenoxy, wobei die cyclischen Reste ihrerseits wieder substituiert sein können durch Alkyl mit 1 bis 6 C-Atomen, Alkoxy mit 1 bis 4 C-Atomen oder Methylendioxy.

$R^2$ einen Alkylrest mit 1 bis 4 C-Atomen oder einen Alkenylrest mit 2 bis 4 C-Atomen, bedeutet,

X einen pflanzenvertraglichen Säurerest bedeutet oder ein pflanzenvertragliches Salz des Piperidinderivats der allgemeinen Formel I.

7. Fungizid gemäß Anspruch 4, dadurch gekennzeichnet, daß $R^2$ Methyl oder Allyl und $X^{\ominus}$ Jodid oder Bromid bedeutet.

## Claims

1. A piperidine derivative of the formula I

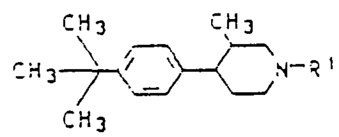

(I)

or or the formula II

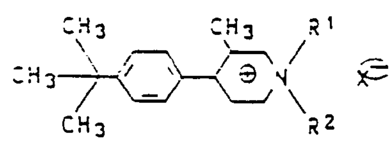

(II)

where $R^1$ is alkyl of 1 to 10 carbon atoms, alkenyl of 2 to 6 carbons, alkynyl of 3 or 4 carbon atoms, and the alkenyl radical may be substituted by phenyl or methoxyphenyl, 1, 2 or 3 $CH_2$ groups in the alkyl radical may be replaced by -O- and the alkyl radical may be substituted by cycloalkyl of 5 to 7 carbon atoms, cycloalkenyl of 5 to 7 carbon atoms, tetrahydropyran, dioxane, bicycloalkyl of 8 to 14 carbon atoms, tricycloalkyl of 8 to 14 carbon atoms, cycloalkoxy of 5 to 7 carbon atoms, phenyl or phenoxy, and the cyclic radicals in turn may be substituted by alkyl of 1 to 6 carbon atoms, alkoxy of 1 to 4 carbon atoms or methylenedioxy, $R^2$ is alkyl of 1 to 4 carbon atoms or alkenyl of 2 to 4 carbon atoms, and X is a plant-tolerated acid radical, and a plant tolerated salt of the piperidine derivative of the formula I.

2. A piperidine derivative as claimed in claim 1, of the formula II where $R^2$ is methyl or allyl and $X^{\ominus}$ is iodine or bromide.

3. A fungicide containing a compound as claimed in claim 1.

4. A fungicide containing a solid, semi-solid or liquid carrier and a piperidine derivative of the formula I

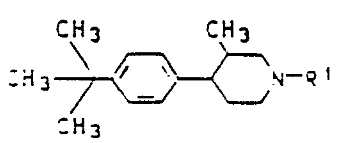

(I)

or of the formula II

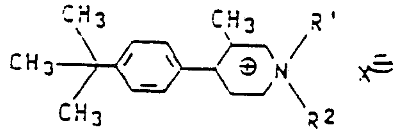

(II)

where $R^1$ is alkyl of 1 to 10 carbon atoms, alkenyl of 2 to 6 carbons, alkynyl of 3 or 4 carbon atoms, and the alkenyl radical may be substituted by phenyl or methoxyphenyl, 1, 2 or 3 $CH_2$ groups in the alkyl radical may be replaced by -O- and the alkyl radical may be substitutedy cycloalkyl of 5 to 7 carbon atoms, cycloalkenyl of 5 to 7 carbon atoms, tetrahydropyran, dioxane, bicycloalkyl of 8 to 14 carbon atoms, tricycloalkyl of 8 to 14 carbon atoms, cycloalkoxy of 5 to 7 carbon atoms, phenyl or phenoxy, and the cyclic radicals in turn may be substituted by alkyl of 1 to 6 carbon atoms, alkoxy of 1 to 4 carbon atoms or methylenedioxy, $R^2$ is alkyl of 1 to 4 carbon atoms or alkenyl of 2 to 4 carbon atoms, and X is a plant-tolerated acid radical, a plant-tolerated salt of the piperidine derivative of the formula I.

5. A process for the preparation of a fungicide, which comprises mixing a solid, semi-solid or liquid carrier with a compound as claimed in claim 1.

6. A method for controlling fungi, except in human or veterinary medicine, wherein the fungi, or the materials, plants, seeds or soil threatened by fungal attack are treated with a piperidine derivative of the formula I

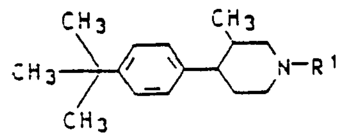

(I)

or of the formula II

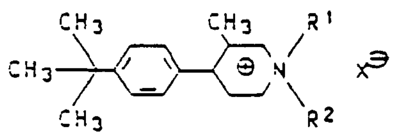

(II)

where $R^1$ is alkyl of 1 to 10 carbon atoms, alkenyl of 2 to 6 carbons, alkynyl of 3 or 4 carbon atoms, and the alkenyl radical may be substituted by phenyl or methoxyphenyl, 1, 2 or 3 $CH_2$ groups in the alkyl radical may be replaced by -O- and the alkyl radical may be substituted by cycloalkyl of 5 to 7 carbon atoms, cycloalkenyl of 5 to 7 carbon atoms, tetrahydropyran, dioxane, bicycloalkyl of 8 to 14 carbon atoms, tricycloalkyl of 8 to 14 carbon atoms, cycloalkoxy of 5 to 7 carbon atoms, phenyl or phenoxy, and the cyclic radicals in turn may be substituted by alkyl of 1 to 6 carbon atoms, alkoxy of 1 to 4 carbon atoms or methylenedioxy, $R^2$ is alkyl of 1 to 4 carbon atoms or alkenyl of 2 to 4 carbon atoms, and X is a plant-tolerated acid radical, a plant-tolerated salt of the piperidine derivative of the formula I.

7. A fungicide as claimed in claim 4, where $R^2$ is methyl or allyl and $X^\ominus$ is iodide or bromide.

**Revendications**

1. Dérivé de pipéridine de formule générale I

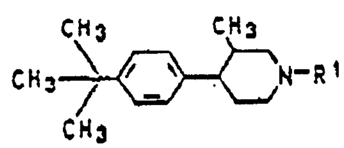

(I)

ou de formule générale II

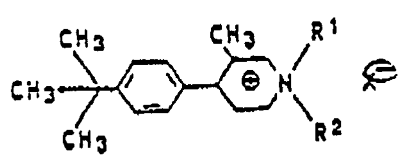

(II)

dans laquelle

$R^1$ représente un reste alkyle à 1 à 10 atomes C, un reste alcényle à 2 à 6 atomes C, un reste alcinyle à 3 à 4 atomes C, le reste alcényle pouvant être substitué par phényle ou méthoxyphényle et, dans le reste alkyle, 1 à 3 groupes $CH_2$ pouvant être remplacés par -O-, et le reste alkyle pouvant être substitué par cycloalkyle à 5 à 7 atomes C, cycloalcényle à 5 à 7 atomes C, tétrahydropyrane, dioxane, bicycloalkyle à 8 à 14 atomes C,

tricycloalkyle à 8 à 14 atomes C, cycloalcoxy à 5 à 7 atomes C, phényle, phénoxy, les restes cycliques de leur côté pouvant être à nouveau substitués par alkyle à 1 à 6 atomes C, alcoxy à 1 à 4 atomes C ou méthylènedioxy,

$R^2$ représente un reste alkyle à 1 à 4 atomes 6 ou un reste alcényle à 2 à 4 atomes C,

X représente un reste acide acceptable pour les plantes, et les sels acceptables pour les plantes du dérivé de pipéridine de formule générale I.

2. Dérivé de pipéridine selon la revendication 1, de formule générale II, dans laquelle $R^2$ représente méthyle ou alkyle et $X^{\ominus}$ représente iodure ou bromure.

3. Fongicide contenant un composé selon la revendication 1.

4. Fongicide contenant un support solide, semi-solide ou liquide et un dérivé de pipéridine de formule générale I

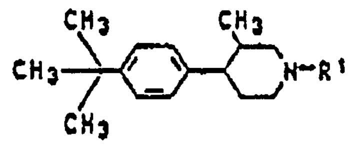

(I)

ou de formule générale II

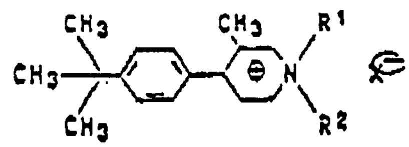

(II)

dans laquelle

$R^1$ représente un reste alkyle à 1 à 10 atomes C, un reste alcényle à 2 à 6 atomes C, un reste alcinyle à 3 à 4 atomes C, le reste alcényle pouvant être substitué par phényle ou méthoxyphényle et, dans le reste alkyle, 1 à 3 groupes $CH_2$ pouvant être remplacés par -O-, et le reste alkyle pouvant être substitué par cycloalkyle à 5 à 7 atomes C, cycloalcényle à 5 à 7 atomes C, tétrahydropyrane, dioxane, bicycloalkyle à 8 à 14 atomes C, tricycloalkyle à 8 à 14 atomes C, cycloalcoxy à 5 à 7 atomes C, phényle, phénoxy, les restes cycliques de leur côté pouvant être à nouveau substitués par alkyle à 1 à 6 atomes C, alcoxy à 1 à 4 atomes C ou méthylènedioxy,

$R^2$ représente un reste alkyle à 1 à 4 atomes C ou un reste alcényle à 2 à 4 atomes C,

X représente un reste acide acceptable pour les plantes, et les sels acceptables pour les plantes du dérivé de pipéridine de formule générale I.

5. Procédé de préparation d'un fongicide, caractérisé par le fait que l'on mélange un support solide, semi-solide ou liquide avec un composé selon la revendication 1.

6. Procédé de lutte contre les champignons, en dehors de la médecine humaine ou vétérinaire, caractérisé par le fait que l'on traite les champignons ou les matériaux, plantes, semences ou sols menacés par l'attaque de champignons, avec un dérivé de pipéridine de formule générale I

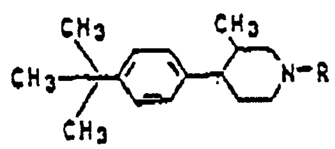

(I)

ou de formule générale II

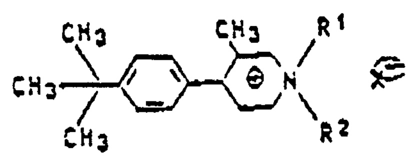

(II)

dans laquelle

$R^1$ représente un reste alkyle à 1 à 10 atomes C, un reste alcényle à 2 à 6 atomes C, un reste alcinyle à 3 à 4 atomes C, le reste alcényle pouvant être substitué par phényle ou méthoxyphényle et, dans le reste alkyle, 1 à 3 groupes $CH_2$ pouvant être remplacés par -O-, et le reste alkyle pouvant être substitué par cycloalkyle à 5 à 7 atomes C, cycloalcényle à 5 à 7 atomes C, tétrahydropyrane, dioxane, bicycloalkyle à 8 à 14 atomes C, tricycloalkyle à 8 à 14 atomes C, cycloalcoxy à 5 à 7 atomes C, phényle, phénoxy, les restes cycliques de leur côté pouvant être à nouveau substitués par alkyle à 1 à 6 atomes C, alcoxy à 1 à 4 atomes C ou méthylènedioxy,

$R^2$ représente un reste alkyle à 1 à 4 atomes C ou un reste alcényle à 2 à 4 atomes C,

X représente un reste acide acceptable pour les plantes, et les sels acceptables pour les plantes du dérivé de pipéridine de formule générale 2.

7. Fongicide selon la revendication 4, caractérisé par le fait que 2 représente méthyle ou alkyle et $X^{\ominus}$ représente iodure ou bromure.